# EUROPEAN PATENT SPECIFICATION

(11) **EP 0 422 166 B1**
(45) Date of publication and mention of the grant of the patent: **01.06.1994**
(21) Application number: 90905982.6
(22) Date of filing: 04.04.1990
(51) Int. Cl.: A61F 5/00

(54) **STATIC SPINAL ALIGNMENT DEVICE**
STATISCHE AUSRICHTANORDNUNG FÜR DEN RÜCKEN
DISPOSITIF D'ALIGNEMENT SPINAL STATIQUE

(30) Priority: 05.04.1989 US 333333
(43) Date of publication of application: 17.04.1991
(73) Proprietor: PACIFIC MEDICAL PRODUCTS, Encino, California 91416-8047 (US)
(72) Inventor: DUMAS, Armen, Oxnard, CA 93035 (US); PLONE, Irving, H., Playa del Rey, CA 90293 (US); ZACUTO, Bradley, J., Van Nuys, CA 91406 (US)
(74) Representative: Silverman, Warren
(86) International application number: US9001810
(87) International publication number: WO9011741

(56) References cited:
- EP-A- 0 123 474
- DE-A- 3 804 056
- GB-A- 2 163 951
- US-A- 744 713
- US-A- 2 056 767
- US-A- 3 811 140
- US-A- 4 230 099

## Description

### BACKGROUND OF THE INVENTION

The present invention relates to a spinal alignment device. In particular it relates to a novel back alignment device which utilizes static side buttressing to straighten the alignment of the spine and further to reduce lumbar lordosis.

In the treatment of back pain, it is necessary to straighten the alignment of the spine while reducing lumbar lordosis. Traction devices have been used for many years in such treatment. A traction device typically flattens the lumbar spine to overcome the anatomical lordosis. In one known traction device, the buttocks of a patient are cradled and lifted so that the patient's own weight provides a traction force to overcome the lumbar lordosis. For example, see U.S.-A-4,362,151. A significant disadvantage and limitation of such a traction device is that it requires the patient to be confined to a bed so that the cradle can be supported by ropes and pulleys with the appropriate counter-balances.

Ambulatory traction devices are also known. For example, in U.S.-A-3,889,664, a surgical brace device for ambulatory treatment of the lower back is disclosed. The device disclosed therein includes a pair of spaced apart torso belt members joined together with jack screw connecting means for applying traction to the user between the pair of belts. The device further includes a pair of adjustably spaced lordosis pads mounted on the lower belt. These pads apply forward pressure on spaced apart back vertebra.

A disadvantage and limitation of each of the above described traction devices is that they are active devices which require trained personnel to position the patient in the device or apply the device around the patient. It is highly desirable to provide a "static" approach wherein the patient can use the device at home without any special expertise or knowledge of device set up.

A typical home remedy for sufferers of back pain is to lie on a floor or some other hard surface to obtain relief. However, while lying on a floor can reduce lumbar lordosis, it cannot ensure alignment of the spine. Reducing lumbar lordosis without spinal alignment can, in fact, cause more pain and damage than relief because of the potential to apply unwanted stress on the joints, increase disc pressure and pinch the nerve roots emanating from the spinal cord.

Static devices for home remedies are also known. For example, in U.S.-A-4,597,386, there is disclosed a lumbar support system which is attached to the backrest of a chair. The lumbar support flexes curvilinearly to match the curve of the lumbar lordosis. A disadvantage and limitation of such a lumbar support is that, while providing lumbar support, it does not straighten the alignment of the vertebra of the spine.

Another device and method for the reduction of lumbar lordosis is described in U.S.-A-4,483,329. This device is positioned between a flat surface and a patient lying supine on the surface. The support device is positioned in the sacral area of the patient to support the apex of the patient's sacrum without providing support for the base of the patient's sacrum. The patient's body weight displaces the sacral base posterially and displaces the apex anteriorly to reduce lumbar lordosis. A disadvantage and limitation of the device described in US-A-4,483,329 patent is that it still does not straighten the alignment of the spinal vertebra.

US-A-4,230,099 (Richardson) discloses a spinal alignment device which comprises two parallel upwardly extending elongated ridges. Each ridge, when viewed perpendicular to the longitudinal axis of the device, has a convex curve corresponding to the lumbar curve of the human spine, a concave curve corresponding to the thoracic curve of the human spine, and a convex curve corresponding to the cervical curve of the human spine. In use, a human body lies supine on the device such that the ridges are positioned adjacent the spine.

According to the present invention, there is provided a spinal alignment device comprising first and second curved portions, and a third portion intermediate the first and second portions, wherein the first, second and third portions form a generally elongate member having an upper surface and a lower surface adapted to support the device on a generally flat surface, characterised in that said upper surface is generally concave in section taken in a plane intersecting the longitudinal axis of the elongate member; and in that the arrangement is such that a human body lying supine on the device is supported by said first and second portions so as to hold the body in a predetermined configuration defined by the curvature of said first and second portions, thereby tending to straighten the spine.

In use, the first and second curved portions are able to buttress a respective side of a patient lying supine on a generally flat surface. The side buttressing maintains the spine of the patient in a spaced relationship to the surface between the thoracic vertebra and a sacrum of the spine and further straightens the alignment of the spine.

A feature of the present invention is that body weight exerts a downward force on the lumbar vertebra to reduce lumbar lordosis while the side buttressing straightens the alignment of the spine. Such a device may, in one embodiment of the present invention, be of unitary construction so that it is small, light and portable enough for use in the home.

For a better understanding of the invention, and to show how the same may be carried into effect, reference will now be made, by way of example, to the accompanying drawings, in which:-
FIG. 1 is a perspective view of a static spinal alignment device constructed according to the principles of the present invention;
FIG. 2 is a cross-sectional view of the static spinal alignment device taken along line II-II of FIG. 1;
FIGS. 3A and 3B exhibit axial spinal misalignment (FIG. 3A) and axial spinal alignment (FIG. 3B) achieved by using the device of FIG. 1 shown in top view; and
FIGS. 4A and 4B illustrate the reduction of lumbar lordosis by use of the device of FIG. 1 shown in cross-section taken along line IV-IV of FIG. 1.

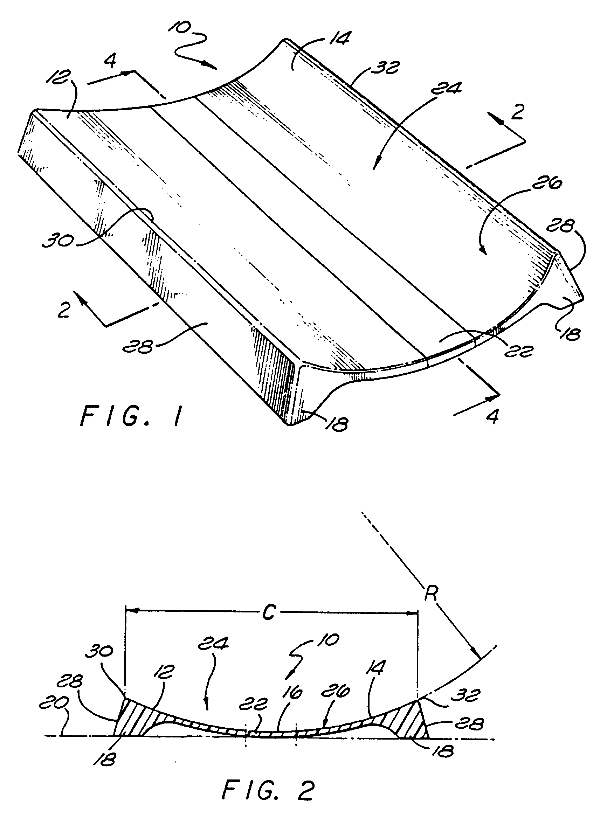

Referring now to FIGS. 1 and 2, there is shown a static or passive spinal alignment device **10** constructed according to the principles of the present invention. The spinal alignment device **10** includes a first elongated section **12** and a second elongated section **14** spaced there-from, means **16** attaching the first section **12** to the second section **14** and means **18** for supporting each of the first section **12** and the second section **14** on a surface **20**.

Attaching means **16** includes a third elongated section **22** co-extensive with and attached to each of the first section **12** and the second section **14** intermediately therewith. Each of the first section **12**, the second section **14**, and the third section **22** form a generally elongated member **24**. Member **24** has an inner surface **26** having a selected radius, R, of curvature as best seen in FIG. 2. Thus, the member **24** is axially elongated and arcuate.

Supporting means **18** includes a pair of axially extending pedestals **28**. Each of the pedestals **28** are co-extensive with and support the respective one of the first section **12** and the second section **14**. An outer surface **30** of the arcuate member **24** is supported by the flat surface **20** axially along the third section **22**.

The static spinal alignment device **10**, as hereinabove described, may be of unitary construction. The axially elongated arcuate member **24** may have a length in a range between 20 inches (50.8 centimeters) and 28 inches (70.1 centimeters). In a preferred embodiment of the present invention, the length of the member **24** is 24 inches (61.0 centimeters). The radius of curvature, R, is in a range between 16 inches (40.6 centimeters) and 20 inches (50.8 centimeters). In a preferred embodiment of the present invention, the radius of curvature, R, is 18 inches (45.7 centimeters). The distance along a cord, C, between an axial lateral edge **30** of the first section **12** and an axial lateral edge **32** of the second section **14** may be in a range between 15 inches (38.1 centimeters) to 17 inches (43.2 centimeters). In a preferred embodiment of the present invention, this distance along the cord, C, between the lateral edge **30** and the lateral edge **32** of the first section **12** and second section **14**, respectively, is 16 inches (40.6 centimeters). Each of the first lateral edge **30** and second lateral edge **32** of the first section **12** and second section **14**, respectively, are identically elevationally spaced from the flat surface **20** in a range of 1.5 inches (3.81 centimeters) to 2.5 inches (6.35 centimeters). In a preferred embodiment of the present invention, these lateral edges **30**, **32** are elevationally spaced from the surface **20** by a distance of 2 incites (5.08 centimeters).

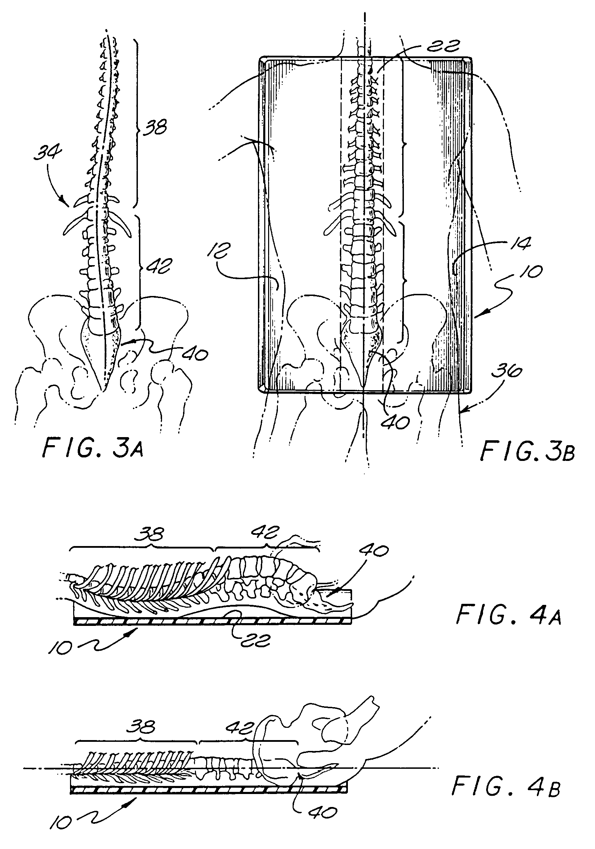

Referring now further to FIGS. 3A and 3B, a spine **34** of a patient **36** is, as best seen in FIG. 3A, shown to be misaligned, such as when the patient **36** may be lying on the flat surface **20**, without using the device **10** of the present invention. With particular reference to FIG. 3B, the first section **12** and the second section **14** of the static spinal alignment device **10** engages each side of the patient **36** lying supine on the flat surface **20** to straighten the alignment of the spine **34**. The static spinal alignment device **10**, and more particularly the first section **12** and second section **14** are positioned to achieve side buttressing of the spine including and between the thoracic vertebra **38** and the sacrum **40** of the spine. As best seen in FIG. 3B, the spine **34** is then straightened and aligned with the third section **22** of the spinal alignment device **10**.

As best seen in FIGS. 4A and 4B, as the patient **36** lies in the static spinal alignment device **10** and is supported by the first section **12** and second section **14**, the lumbar vertebra **42** between the thoracic vertebra **38** and sacrum **40** are spaced from the third section **22**. In FIG. 4A, the lumbar vertebra **42** are shown with an extreme lumbar lordosis. As best seen in FIG. 4B, the body weight of the patient **36** reduces the lumbar lordosis, while maintaining the spine in alignment from the side buttressing as described hereinabove. The side buttressing provided by the first section **12** and second section **14** exerts pressure on the patient laterally inwardly in response to the body weight of the patient to maintain the alignment of the spine **34**. The body weight of the patient urges the straightened spine **34** downwardly to reduce the lumbar lordosis.

The spinal alignment device in accordance with the present invention can simultaneously straighten the alignment of the spine and reduce the lumbar lordosis.

## Claims

1. A spinal alignment device (10), comprising:
first (12) and second (14) curved portions, and
a third portion (22) intermediate the first (12) and second (14) portions,
wherein the first (12), second (14) and third (22) portions form a generally elongate member (24) having an upper surface (26) and a lower surface adapted to support the device (10) on a generally flat surface (20),
characterised in that said upper surface (26) is generally concave in section taken in a plane intersecting the longitudinal axis of the elongate member (24); and in that the arrangement is such that a human body (36) lying supine on the device (10) is supported by said first (12) and second (14) portions so as to hold the body (36) in a predetermined configuration defined by the curvature of said first (12) and second (14) portions, thereby tending to straighten the spine (34).

2. A spinal alignment device (10) as claimed in claim 1, wherein the upper surface (26) has a radius of curvature (R) selected to maintain the spine (34) in a spaced relationship to the third portion (22).

3. A spinal alignment device (10) as claimed in claim 2, wherein the radius of curvature (R) of the upper surface (26) is in the range 40 to 51 cm.

4. A spinal alignment device (10) as claimed in claim 3, wherein the radius of curvature (R) of the upper surface (26) is about 46 cm.

5. A spinal alignment device (10) as claimed in claim 1, 2, 3 or 4, having an axial length such that, in use, it extends between the thoracic vertebra (38) and the sacrum (40) of the spine (34).

6. A spinal alignment device (10) as claimed in claim 5, wherein the axial length is in the range 50 to 70 cm.

7. A spinal alignment device (10) as claimed in claim 6, wherein the axial length is about 61 cm.

8. A spinal alignment device (10) as claimed in any preceding claim, wherein said first (12) and second (14) portions each have an axially-extending co-extensive pedestal (28) for supporting the device (10) on said generally flat surface (20).

9. A spinal alignment device (10) as claimed in any preceding claim, wherein, in use, the distance between axially-extending lateral edges (30,32) of the first (12) and second (14) portions and said generally flat surface (20) is in the range 3.8 to 6.4 cm.

10. A spinal alignment device (10) as claimed in claim 9, wherein the distance between axially-extending lateral edges (30,32) of the first (12) and second (14) portions and said generally flat surface (20) is about 5 cm.

11. A spinal alignment device (10) as claimed in claim 9 or 10, wherein the distance along a cord (C) between the lateral edges (30,32) of the first (12) and second (14) portions is in the range 38 to 43 cm.

12. A spinal alignment device (10) as claimed in claim 11, wherein the distance along a cord (C) between the lateral edges (30,32) of the first (12) and second (14) portions is about 41 cm.

13. A spinal alignment device (10) as claimed in any preceding claim, wherein the device is of unitary construction.

## Patentansprüche

1. Vorrichtung zur Ausrichtung eines Rückgrates (10), umfassend:
erste (12) und zweite (14) gebogene Abschnitte und einen dritten Abschnitt (22) zwischen den ersten (12) und zweiten (14) Abschnitten,
worin die ersten (12), zweiten (14) und dritten (22) Abschnitte ein im allgemeinen ausgedehntes Element (24) bilden, mit einer oberen Oberfläche (26) und einer unteren Oberfläche, die angepaßt ist, um die Vorrichtung (10) auf einer im allgemeinen flachen Oberfläche (20) zu halten,
**dadurch gekennzeichnet, daß**
die genannte obere Oberfläche (26) in einem Schnitt, ausgeführt durch eine Ebene, welche die Längsachse des ausgedehnten Elements (24) schneidet, im allgemeinen konkav ist;
und dadurch, daß die Anordnung solchermaßen ist, daß ein menschlicher Körper (36), der auf dem Rücken auf der Vorrichtung (10) liegt, durch die genannten ersten (12) und zweiten (14) Abschnitte unterstützt wird, um so den Körper (36) in einer vorbestimmten Lage, welche durch die Krümmung der genannten ersten (12) und zweiten (14) Abschnitte definiert ist, zu halten, was dadurch zu einer geraden Ausrichtung der Wirbelsäule (34) führt.

2. Vorrichtung zur Ausrichtung eines Rückgrates (10) gemäß Anspruch 1, worin die obere Oberfläche (26) einen Krümmungsradius (R) hat, welcher so ausgewählt ist, daß die Wirbelsäule (34) in einer Abstandsbeziehung (spaced relationship) zu dem dritten Abschnitt (22) gehalten wird.

3. Vorrichtung zur Ausrichtung eines Rückgrates (10) gemäß Anspruch 2, worin der Krümmungsradius (R) der oberen Oberfläche (26) in dem Bereich von 40 bis 51 cm liegt.

4. Vorrichtung zur Ausrichtung eines Rückgrates (10) gemäß Anspruch 3, worin der Krümmungsradius (R) der oberen Oberfläche (26) ca. 46 cm beträgt.

5. Vorrichtung zur Ausrichtung eines Rückgrates (10) gemäß Anspruch 1, 2, 3 oder 4, mit einer axialen Länge, die solchermaßen ist, daß sie sich bei Verwendung zwischen den Brustwirbeln (38) und dem Kreuzbein (40) der Wirbelsäule (34) ausdehnt.

6. Vorrichtung zur Ausrichtung eines Rückgrates (10) gemäß Anspruch 5, worin die axiale Länge in dem Bereich von 50 bis 70 cm liegt.

7. Vorrichtung zur Ausrichtung eines Rückgrates (10) gemäß Anspruch 6, worin die axiale Länge ca. 61 cm beträgt.

8. Vorrichtung zur Ausrichtung eines Rückgrates (10) gemäß irgendeinem der vorhergehenden Ansprüche, worin die genannten ersten (12) und zweiten (14) Abschnitte jeder einen sich axial ausdehnenden koextensiven Sockel (28) haben, um die Vorrichtung (10) auf der genannten im allgemeinen flachen Oberfläche (20) zu unterstützen.

9. Vorrichtung zur Ausrichtung eines Rückgrates (10) gemäß irgendeinem der vorhergehenden Ansprüche, worin, bei Verwendung, der Abstand zwischen den sich axial ausdehnenden Seitenkanten (30, 32) der ersten (12) und zweiten (14) Abschnitte und der genannten im allgemeinen flachen Oberfläche (20) in dem Bereich von 3,8 bis 6,4 cm liegt.

10. Vorrichtung zur Ausrichtung eines Rückgrates (10) gemäß Anspruch 9, worin der Abstand zwischen den sich axial ausdehnenden Seitenkanten (30, 32) der ersten (12) und zweiten (14) Abschnitte und der genannten im allgemeinen flachen Oberfläche (20) ca. 5 cm beträgt.

11. Vorrichtung zur Ausrichtung eines Rückgrates (10) gemäß Anspruch 9 oder 10, worin der Abstand entlang einer Spannweite (C) zwischen den Seitenkanten (30, 32) der ersten (12) und zweiten (14) Abschnitte in dem Bereich von 38 bis 43 cm liegt.

12. Vorrichtung zur Ausrichtung eines Rückgrates (10) gemäß Anspruch 11, worin der Abstand entlang einer Spannweite (C) zwischen den Seitenkanten (30, 32) der ersten (12) und zweiten (14) Abschnitte ca. 41 cm beträgt.

13. Vorrichtung zur Ausrichtung eines Rückgrates (10) gemäß irgendeinem der vorhergehenden Ansprüche, worin die Vorrichtung einen einstückigen Aufbau aufweist.

## Revendications

1. Dispositif d'alignement rachidien (10), comprenant :
une première (12) et une deuxième (14) partie incurvées et une troisième partie (22) intercalée entre la première (12) et la deuxième (14) partie,
dans lequel la première (12), la deuxième (14) et la troisième (22) partie forment un élément généralement allongé (24) présentant une surface supérieure (26) et une surface inférieure conçue pour porter le dispositif (10) sur une surface généralement plane (20),
caractérisé en ce que ladite surface supérieure (26) est de section généralement concave, considérée dans un plan coupant l'axe longitudinal de l'élément allongé (24); et en ce que la disposition est telle qu'un corps humain (36) reposant en décubitus dorsal sur le dispositif (10) est soutenu par lesdites première (12) et deuxième (14) parties, de sorte que le corps (36) est maintenu dans une configuration prédéterminée définie par la courbure desdites première (12) et deuxième (14) parties, tendant ainsi à rectifier l'alignement de la colonne vertébrale (34).

2. Dispositif d'alignement rachidien (10) selon la revendication 1, dans lequel la surface supérieure (26) présente un rayon de courbure (R) sélectionné de façon à maintenir la colonne vertébrale (34) à distance de la troisième partie (22).

3. Dispositif d'alignement rachidien (10) selon la revendication 2, dans lequel le rayon de courbure (R) de la surface supérieure (26) est compris entre 40 et 51 cm.

4. Dispositif d'alignement rachidien (10) selon la revendication 3, dans lequel le rayon de courbure (R) de la surface supérieure (26) est d'environ 46 cm.

5. Dispositif d'alignement rachidien (10) selon la revendication 1, 2, 3 ou 4, ayant une longueur axiale telle que lors de l'emploi il s'étend entre les vertèbres dorsales (38) et le sacrum (40) de la colonne vertébrale (34).

6. Dispositif d'alignement rachidien (10) selon la revendication 5, dans lequel la longueur axiale est comprise entre 50 et 70 cm.

7. Dispositif d'alignement rachidien (10) selon la revendication 6, dans lequel la longueur axiale est d'environ 61 cm.

8. Dispositif d'alignement rachidien (10) selon l'une quelconque des revendications précédentes, dans lequel lesdites première (12) et deuxième (14) parties présentent chacune un socle (28) de même dimension, s'étendant axialement, destiné à porter le dispositif (10) sur ladite surface généralement plane (20).

9. Dispositif d'alignement rachidien (10) selon l'une quelconque des revendications précédentes, dans lequel, lors de l'emploi, la distance entre les arêtes latérales (30, 32), s'étendant axialement, de la première (12) et la deuxième (14) partie et ladite surface généralement plane (20) est comprise entre 3,8 et 6,4 cm.

10. Dispositif d'alignement rachidien (10) selon la revendication 9, dans lequel la distance entre les arêtes latérales (30, 32), s'étendant axialement, de la première (12) et deuxième (14) partie et ladite surface généralement plane (20) est d'environ 5 cm.

11. Dispositif d'alignement rachidien (10) selon la revendication 9 ou 10, dans lequel la longueur d'une corde (C) entre les arêtes latérales (30, 32) de la première (12) et de la deuxième (14) partie est comprise entre 38 et 43 cm.

12. Dispositif d'alignement rachidien (10) selon la revendication 11, dans lequel la longueur d'une corde (C) entre les arêtes latérales (30, 32) de la première (12) et de la deuxième (14) partie est d'environ 41 cm.

13. Dispositif d'alignement rachidien (10) selon l'une quelconque des revendications précédentes, dans lequel le dispositif est monobloc.
